# EUROPÄISCHE PATENTSCHRIFT

(11) **EP 3 233 177 B1**
(45) Veröffentlichungstag und Bekanntmachung des Hinweises auf die Patenterteilung: **10.10.2018**
(21) Anmeldenummer: 15790037.4
(22) Anmeldetag: 27.10.2015
(51) Int. Cl.: A61N 1/04, A61N 1/36

(54) **EMS-REIZSTROMÜBERTRAGUNGSELEMENT, SOWIE MIT DEM EMS-REIZSTROMÜBERTRAGUNGSELEMENT AUSGESTATTETES EMS-KLEIDUNGSSTÜCK**
EMS STIMULATION CURRENT TRANSMISSION ELEMENT, AND EMS GARMENT EQUIPPED WITH THE EMS STIMULATION CURRENT TRANSMISSION ELEMENT
ÉLÉMENT DE TRANSMISSION DE COURANT DE STIMULATION POUR STIMULATION MUSCULAIRE ÉLECTRIQUE (EMS) AINSI QUE VÊTEMENT DE STIMULATION MUSCULAIRE ÉLECTRIQUE ÉQUIPÉ DE CET ÉLEMENT DE TRANSMISSION DE COURANT DE STIMULATION POUR EMS

(30) Priorität: 18.12.2014 DE 102014018683
(43) Veröffentlichungstag der Anmeldung: 25.10.2017
(73) Patentinhaber: Miha Bodytec GmbH, 86368 Gersthofen (DE)
(72) Erfinder: DECKER, Jürgen, 86494 Emersacker (DE)
(74) Vertreter: Munk, Ludwig Hubert
(86) Internationale Anmeldenummer: PCT/EP2015/025074
(87) Internationale Veröffentlichungsnummer: WO 2016/096152

(56) Entgegenhaltungen:
- EP-A1- 0 128 103
- US-A- 3 610 250
- US-A1- 2002 077 688
- US-A1- 2002 077 689

## Beschreibung

Die Erfindung betrifft ein EMS-Reizstromübertragungselement gemäß dem Oberbegriff des Anspruchs 1, sowie ein mit dem EMS-Reizstromübertragungselement ausgestattetes EMS-Kleidungsstück gemäß dem Oberbegriff des Anspruchs 17.

Mit Elektromuskelstimulation (EMS), teilweise auch Elektromyostimulation genannt werden Muskeln im lebenden Körper, im Regelfall zu Muskelaufbauzwecken, beispielsweise in Fitness-Studios oder bei Personal-Trainern mit elektrischen Reizen beaufschlagt, um die Muskeln zu kräftigen. Zu der Erzeugung dieser EMS-Reize bzw. des EMS-Reizstroms werden EMS-Reizerzeugungseinrichtungen eingesetzt, die in der Regel einen elektrischen Impulsgenerator aufweisen, sowie eine elektronische Steuerung.

Die Steuerung gibt ein Anregungsschema vor, dem folgend aus einem von einer Stromquelle bezogenen Strom eine zeitlich und auf die EMS-Elektroden des EMS-Trainingsgerätes verteilte Mehrzahl von EMS-Reizen geformt wird, die aus den Stromimpulsen und/oder Wechselstrom mit per Steuerung vorgegebenen Werten wie Amplitude und Frequenz bestehen, und mit denen am Körper angelegte EMS-Elektroden beaufschlagt werden, um durch den Körper Strom mit vorgegebener Amplitude und Frequenzmuster durchzuleiten. Dabei sind die EMS-Elektroden an dem EMS-Kleidungsstück üblicherweise in Paaren gruppiert, so dass zwei zu einem solchen Paar gruppierte EMS-Elektroden jeweils über einen Leitungsast an der EMS-Reizerzeugungseinheit angeschlossen sind, wobei die beiden Leitungsäste während des EMS-Trainings durch den Körper, an dem ihre EMS-Elektroden anliegen, zu einem geschlossenen Stromkreis vervollständigt werden, der durch den Körper in einem gewünschten Bereich hindurch führt, etwa durch einen Oberschenkel oder Oberarm, und somit dort die Muskeln zu Kontraktionen zwingt und somit kräftigt.

Es werden also in der EMS-Reizerzeugungseinheit getaktete Impulsströme erzeugt und über Stromleitungen zu EMS-Elektroden und über die EMS-Elektroden durch den Körper hindurchgeleitet. Dabei werden im Regelfall die die EMS-Elektroden paarweise so geschaltet, dass bei einem Zeitpunkt eine erste EMS-Elektrode mit dem Strom impulsartig beaufschlagt wird und eine zweite EMS-Elektrode zu diesem Zeitpunkt nicht und den durch den Körper geleiteten Strom abfließen lässt. Beim Impulswechsel wird die zweite EMS-Elektrode mit Strom impulsartig beaufschlagt und die erste EMS-Elektrode nicht. Der Stromlauf ist bei einer Bekleidung mit mehreren EMS-Elektrodenpaaren z.B. Brust, Bauch, Rücken Oberarme, Oberschenkel etc. noch komplexer.

Die EMS-Elektroden sind mit der EMS-Reizerzeugungseinheit, also einem Steuergerät verschaltet, das beispielsweise in einem Frequenzbereich von 2 bis 150 Hz mit einer Impulsbreite von 50 bis 400 Mikrosekunden und einer Impulspause von 0 bis 10 Sekunden arbeitet. Der max. Scheitelwert der elektrischen Ausgangsspannung liegt beispielsweise bei 70 bis 160 Volt bei einer Stromstärke von ca. 10 bis 20 Milliampere.

Dabei wurden früher Polymer-Pads, die mit leitfähigen Partikeln (z.B. Ruß) gefüllt sind, als am Körper anzulegenden EMS-Elektroden eingesetzt. Aus der Diathermie sind ferner Elektroden bekannt, die einen Stromübertragungsbereich bzw. eine Leitschicht aus Metallfolie aufweisen, der mit Kunststoff hinterlegt ist, siehe die deutsche Patentschrift DE 20 18 239 C2.

Derartige EMS-Elektroden sind für heutige EMS-Anwendungen jedoch nicht flexibel und elastisch genug und lassen sich nur schwer in textile Träger integrieren. Denn EMS-Elektroden sind in der Regel an einem am Körper anlegbaren Elektrodenträger angebracht. Während frühere Elektrodenträger häufig aus Riemen- und Lederbändern bestanden, siehe beispielsweise DE 10 2005 058 850 A1, an denen die Elektroden angebracht waren und die Anforderungen an die Elektroden hinsichtlich Flexibilität, Elastizität gering waren, geht die Entwicklung immer mehr hin zu textilen EMS-Kleidungsstücken, die die Elektroden tragen bzw. in die die Elektroden integriert sind.

Derartige EMS-Kleidungsstücke können von der trainierenden Person eben wie ein Kleidungsstück getragen werden, also beispielsweise als Weste, Hose, Strumpf, Armband oder dergleichen und werden meistens vor dem Training genässt oder über einem genässten Unterbekleidungsstück, z.B. T-Shirt getragen, wobei die EMS-Elektroden in der Regel über oder hinter dem eigentlichen Stromübertragungsbereich bzw. ihrer Leitschicht weitere Strukturen aufweisen können, die als Nässespeicher dienen. Es gibt auch textile EMS-Elektroden, bei denen der Stromübertragungsbereich mit dem Nässespeicher in einer gemeinsamen textilen Struktur zusammengefasst ist, wie beispielsweise der deutschen Patentanmeldung DE 10 2007 046 886 A1 zu entnehmen ist. Die dort beschriebenen, am Körper anzulegenden EMS-Elektroden weisen Textilstrukturen mit eingearbeiteten Silberfäden auf. Weitere EMS-Kleidungsstücke sind in den Schriften EP 0 128 103A1, US 3,610,250 A, US 2002/0077689 A1 sowie US2002/0077688 A1 gezeigt.

Neben solchen, externen und am als Träger fungierenden EMS-Kleidungsstück zu befestigenden EMS-Elektroden wurden aus dem Bereich der Thrombose- und Elektromassagestrümpfe und dergleichen bereits textilintegrierte Elektroden bekannt, die in das Gewebe bzw. Gestrick oder dergl. des EMS-Kleidungsstücks bereits miteingewebt bzw. -gestrickt oder dergleichen sind.

So offenbart die deutsche Gebrauchsmusterschrift DE 202 09 219 U1 ein Gestrick für einen Strumpf, mit als Elektroden dienenden Bereichen, in denen leitfähige Fäden eingestrickt sind, die an definierten Endpunkten Anschlußmöglichkeiten für eine Meß- oder Versorgungsspannung aufweisen. Diese leitfähigen Fäden sind silberbeschichtete Multifilamentfäden oder litzenartige Metallfäden. Der Anschluss an die Stromversorgung erfolgt über leitfähige Druckknopf-Verbindung, wobei die freien Enden des leitfähigen Fadens mit Hilfe eines in das Gestrick eingearbeiteten und anschließend erschmolzenen Schmelzfadens gebunden werden.

Derartige Elektroden werden an den Druckknöpfen an die Stromversorgung bzw. im Fall von EMS-Elektroden an die EMS-Reizerzeugungseinheit angeschlossen. EMS-Kleidungsstücke mit mehreren EMS-Elektroden erhalten so ein für den Verbraucher unattraktives, weil stark verkabeltes Äußeres. Zudem neigen die Druckknöpfe unerklärlich und örtlich und zeitlich zufällig verteilt zu Korrosion.

Aus dem Elektromassagebereich bekannte Konzepte für Elektroden, die integral mit den Zuleitungen als leitfähige Bereiche in das Kleidungsstück eingewebt sind und aus einer flexiblen, leitfähigen Faser bestehen, siehe beispielsweise die in der deutschen Gebrauchsmusterschrift DE 20 2004 004 582 U1 und der internationalen Patentanmeldung WO2011/118918 A2 gezeigten Massagehandschuhe und -socken, wurden adaptiert, um die sichtbaren Kabel an EMS-Kleidungsstücken zu eliminieren oder zumindest auf die von dem EMS-Kleidungsstück zu einer externen Steuereinheit führenden Kabel zu verkürzen.

Heutzutage gibt es daher auch textilintegrierte EMS-Elektroden, die insgesamt oder zumindest deren Stromübertragungsbereich in das Trägertextil eingearbeitet sind, wobei neben den Elektroden auch die an die Elektroden angeschlossenen Leiterbahnen in das EMS-Kleidungsstück integriert sind.

So offenbart die deutsche Gebrauchsmusterschrift DE 20 2011 109 226 U1 ein EMS-Kleidungsstück mit textilen Kontaktelektroden bzw. EMS-Stromübertragungsbereichen, die über in das Kleidungsstück eingearbeitete, ebenfalls textilbasierte Zuleitungen jeweils mit einem zugeordneten Anschlusselement in Form eines Druckknopfs oder dergleichen verbunden sind. Die Kontaktelektroden bzw. Stromübertragungsbereiche können dabei aus einem Gewebe aus Silberfäden und Elasthan oder aus einem auf ein Trägermaterial aufgebrachten Silbergewirk. Während Gewebe aus einer Vielzahl von Fäden, nämlich Kettfäden und Schuss besteht zählen Gewirke zu den Maschenwaren.

Weiterhin zeigen die deutsche Patentanmeldung DE 10 2012 112 153 A1 und die internationale Patentanmeldung WO 2014/000736A2 textilintegrierte EMS-Elektroden, deren Stromübertragungsbereich als Inselintarsie in ein Flachgestrick eingearbeitet und durch eine ebenfalls eingestrickte Anschlussleitung, die in einem ebenfalls eingestrickten Kanal verläuft, mit einem Stromanschluss verbunden ist. Die Inselintarsien weisen zur Haut des Nutzers hin eine Implantierung auf, welche bevorzugt aus Kautschuk besteht. Hierzu sei angemerkt, dass eine Intarsie entsteht, indem innerhalb einer Maschenreihe einzelne Abschnitte der Reihe mit unterschiedlichen Garnen hergestellt werden (wie etwa bei mehrfarbigen Norwegerpullovern). Die Inselintarsien sind also durch Maschen des Flachgestricks gebildet.

Die internationale Patentanmeldung WO 2004 006 700 A1 zeigt ein Rundgestrick mit eingestrickten Elektroden, die zusammen mit den Zuleitungen eingestrickt sein können. Die Stromübertragungsbereiche der EMS-Elektroden, aber auch die Zuleitungen werden also jeweils durch einen zu ineinander verschlungenen Maschen verstrickten Faden gebildet, der von Maschenreihe zu Maschenreihe linienförmige Stromleiterstrangabschnitte bildet, entlang denen der Stromfluss erfolgt. Der Stromfluss folgt also nicht dem Verlauf des Fadens, sondern springt von Maschenreihe zu Maschenreihe von einem linienförmige Stromleiterstrangabschnitt des Fadens zu einem anderen linienförmige Stromleiterstrangabschnitt des Fadens, welche Stromleiterstrangabschnitt in dem durch den Faden gebildeten Strang nicht aneinander anschließen.

Derartige EMS-Kleidungsstücke mit textilintegrierten EMS-Elektroden sind zwar möglicherweise in einem Arbeitsgang fertigbar, was aber durchaus nicht ganz unkompliziert ist und im Textilfertigungsprozess Herausforderungen birgt. Ferner liegt bei derartigen, kleidungsintegrierten EMS-Elektroden ein besonderer Augenmerk darauf, dass sie eine hohe Flexibilität und möglichst auch Elastizität aufweisen, da die EMS-Kleidungsstücke und mit ihnen die EMS-Elektroden eng am Körper anliegen müssen. Diese Flexibilität und Elastizität lässt sich mit den für die Gewebe und Gestricke verwendeten, leitfähigen Fäden nicht oder mit teuren Spezialfäden, die gleichzeitig leitfähig und elastisch sind, nur sehr kostspielig erreichen.

Hiervon ausgehend ist es Aufgabe der vorliegenden Erfindung, ein EMS-Reizstromübertragungselement sowie ein mit dem EMS-Reizstromübertragungselement ausgestattetes EMS-Kleidungsstück zu schaffen, die robust sind und über einen langen Zeitraum funktionsfähig bleiben, sowie bei hoher Flexibilität und Elastizität kostengünstig zu fertigen sind.

Diese Aufgabe wird hinsichtlich des EMS-Reizstromübertragungselement mit den Merkmalen des Anspruchs 1 gelöst und hinsichtlich des mit dem EMS-Reizstromübertragungselement ausgestatteten EMS-Kleidungsstücks mit den Merkmalen des Anspruchs 17.

Ein gattungsgemäßes EMS-Reizstromübertragungselement für ein EMS-Kleidungsstück weist zumindest einen flächenförmigen Stromübertragungsbereich einer EMS-Elektrode zur Übertragung von EMS-Reizen auf den lebenden Körper auf. Der Stromübertragungsbereich enthält eine Anzahl flächig angeordneter, linienförmiger Stromleiterstrangabschnitte und ist über eine weitere Anzahl linienförmiger Stromleiterstrangabschnitte an Anschlussstelle angeschlossen, die in der Regel vom Stromübertragungsbereich beabstandet ist. Die Stromleiterstrangabschnitte sind dabei zwar linienförmig im Gegensatz zu einem flächigen Element wie einer Leiterfolie, müssen aber nicht zwangsläufig entlang gerader Linien verlegt sein. An der Anschlussstelle ist das EMS-Reizstromübertragungselement an eine EMS-Reizerzeugungseinheit anschließbar, um den Stromübertragungsbereich mit dem von der EMS-Reizstromerzeugungseinheit aus einem von einer Stromquelle bezogenen Strom zu einer Impulsfolge und/oder zu einem Wechselstrom geformten EMS-Reizstrom zu beschicken.

Erfindungsgemäß weist der Stromübertragungsbereich des EMS-Reizstromübertragungselements einen einzigen, linienförmigen Stromleiterstrangabschnitt auf, der flächig und ohne Maschen verlegt ist. Auf der Fläche des Stromübertragungsbereich ist also ein linienförmiger Stromleiterstrangabschnitt so verlegt, dass er die Fläche bedeckt. Der linienförmige Stromleiterstrangabschnitt wird mitsamt einem zur Anschlussstelle führenden, linienförmigen Stromleiterstrangabschnitt von einem einzigen, linienförmigen Stromleiterstrang gebildet. Der Stromfluss folgt also dem linienförmigen Stromleiterstrang und springt nicht von Masche zu Masche bzw. im Gewebe. Das erfindungsgemäße EMS-Kleidungsstück weist zumindest ein solches EMS-Reizstromübertragungselement auf.

Der Erfindung liegt die Erkenntnis zugrunde, dass systemische Fehler an den Verbindungsstellen der Stromübertragungsbereiche mit den Zuleitungen, zu unterschiedlichen Widerständen in den beiden einander zugeordneten Leitungsästen führen können. Denn die Verbindungsstellen sind häufig als Druckknöpfe oder Crimp-Stecker ausgebildet oder als nicht oder nicht vollständig miteinander vermaschte Gestricke. Bei der Fertigung kann es vorkommen, dass die mechanische Verbindung nicht einwandfrei hergestellt wird, was dazu führt, dass an der betroffenen Verbindungsstelle in elektrischer Hinsicht ein Zwischenwiderstand entsteht, im gegenüberliegenden, zugeordneten Leitungsast aber möglicherweise nicht. Aber auch ein Gewebe bzw. Gestrick selbst ist anfällig für Maschenfehler oder für aufgrund Abnutzung auftretende Ausrisse im Gewebe bzw. Gestrick, die ebenfalls zu einem Zwischenwiderstand und somit zu unterschiedlichen Widerständen in den beiden einander zugeordneten Leitungsästen führen können.

Durch diese Widerstandsunterschiede kommt es zu unterschiedlichen Strömen im Strombeaufschlagungsleitungsast gegenüber dem Stromableitungsleitungsast, was zu einer Elektronenabgabe beispielsweise des Elektrodenmaterials, des Elektroden-/Stromleitungskontaktierungsmaterials oder in der Stromleitung und damit letztlich zu Oxidation führt. Diese an unterschiedlichen Stellen im Stromkreis auftretende Oxidation und damit Korrosion, bei der an den Korrosionsstellen unter Elektronenabgabe das dort vorhandene metallische Material oxidiert und damit korrodiert, wirkt nun selbst so, als ob an den korrodierenden Stellen in elektrischer Hinsicht ein Zwischenwiderstand eingebaut würde. Mit der Zeit kann die Oxidation und damit Widerstandserhöhung soweit gehen, dass die im betroffenen Leitungsast angeordnete EMS-Elektrode keinen für die gewünschte Muskelkontraktion nötigen EMS-Reiz mehr übertragen kann. Durch den Schweiß bzw. die elektrolytische Wirkung des Schweißes wird dieser Vorgang verstärkt und beschleunigt.

Durch die erfindungsgemäße Ausbildung der EMS-Elektrode bzw. deren Stromübertragungsbereichs mitsamt Zuleitung durch einen einzigen Stromleiterstrang wird eine solche fehleranfällige Verbindungsstelle, sei sie als Druckknöpfe/Crimp-Stecker oder als Verbindung zweier Gestricke ausgeführt, beseitigt, so dass eine große Quelle für Fehler im Fertigungsprozess vermieden wird. Zudem kann durch die unvermaschte Verlegung des Stromleiterstrangs auf einer Fläche eine gegenüber einer Maschenware oder einem Gewebe eine detulich höhere Elastizität des Gesamtelements erzielt werden. Weiterhin gestaltet sich auch die Fertigung eines solchen EMS- Reizstromübertragungselements recht einfach und kostengünstig, wie weiter unten noch erläutert wird.

Gemäß eines alternativen Aspekts der Erfindung könnten anstatt einem einzigen Stromleiterstrang auch mehrere, parallel mit Strom versorgbare Stromleiterstränge vorgesehen sein. Der Stromübertragungsbereich weist also eine Mehrzahl parallel verlegter, linienförmigen Stromleiterstrangabschnitte auf, die jeweils ohne Maschen flächig verlegt sind und die jeweils mit einem der zur Anschlussstelle führenden Stromleiterstrangabschnitte von einem einzigen, linienförmigen Stromleiterstrang gebildet werden.

Gemäß eines weiteren alternativen Aspekts der Erfindung könnte der Stromübertragungsbereich ferner eine Mehrzahl parallel und ohne Maschen flächig verlegter, linienförmiger Stromleiterstrangabschnitte aufweisen, die alle mit dem einzigen, linienförmigen Stromleiterstrangabschnitt verspleißt sind, der zur Anschlussstelle führt.

Sollte neben der Anschlussstelle für die Stromzufuhr noch eine Masseanschlussstelle nötig sein, um einen bei der Beaufschlagung des Körpers mit dem EMS-Reizstrom auf den Stromübertragungsbereich übertragenen Strom abzuleiten, oder eine weiter Anschlussstelle, um dem Stromübertragungsbereich parallel von beiden Enden des Stromleiterstrangs Strom zuzuführen, so könnte der Stromleiterstrangabschnitt von der weiteren Anschlussstelle zum Stromübertragungsbereich analog zum Stromleiterstrangabschnitt von der Anschlussstelle zum Stromübertragungsbereich integral und einstückig an dem einzigen Stromleiterstrang ausgebildet sein, der von der Anschlussstelle zum Stromübertragungsbereich, durch diesen hindurch und dann zur weiteren Anschlussstelle führt. Mehrere parallele Stromleiterstränge und ein Wiederzusammenspleißen von im Stromübertragungsbereich aufgespleißten Stromleiterstrangabschnitten wären ebenfalls denkbar.

Um die Stromleiterstrangabschnitte, gemäß des bevorzugten ersten Aspekts der Erfindung den einzigen Stromleiterstrang im Stromübertragungsbereich flächig zu verlegen ist es vorteilhaft, wenn das EMS-Reizstromübertragungselement ein Trägerelement aufweist, auf dem die Stromleiterstrangabschnitte, gemäß des bevorzugten Aspekts der Erfindung den einzigen Stromleiterstrang angeordnet sind bzw. ist. Das Trägerelement ist dabei bevorzugt als textiles Flächengebilde ausgebildet. Je nach gewünschter Eigenschaft des EMS-Reizstromübertragungselement kann das Trägerelement elastisch, unelastisch sowie isolierend bzw. ein-, oder beidseitig mit einer Isolierschicht versehen oder unisoliert sein. Das Trägerelement kann vorteilhaft auch hoch waschfest und flammhemmend sein.

Die Stromleiterstrangabschnitte bzw. der einzige Stromleiterstrang können bzw. kann dann zumindest im Bereich des Stromübertragungsbereichs auf das Trägerelement mittels eines weiteren Fadens aufgenäht oder aufgestickt sein. Hierbei kann die Stichlänge variieren, um an dem Stromleiterstrang bzw. den Leiterbahnen mehr oder weniger Flexibilität zu erreichen. Es wäre aber auch denkbar, die Stromleiterstrangabschnitte bzw. den einzige Stromleiterstrang selbst als das Nähgarn für die Naht zu verwenden und so in das textile Trägerelement gewissermaßen einzunähen oder zu -sticken.

Das textile Trägerelement kann dabei gleich als EMS-Kleidungsstück geformt sein, also beispielsweise als Weste oder Hose. Vorteilhaft ist EMS-Reizstromübertragungselement aber mit einem Trägerelement versehen, an dem es in ein Kleidungsstück eingenäht werden kann. Das Trägerelement kann dabei etwa mittels Laserschneidens entlang des bzw. der darauf verlegten Stromleiterstränge, also um den bzw. die Stromübertragungsbereiche und die Zuleitungen herum aus einer Stoffbahn oder dergleichen herausgeschnitten sein.

Das Trägerelement des EMS-Reizstromübertragungselements kann dabei vorteilhaft nicht oder nur schlecht elektrisch leitend sein, insbesondere auch im genässten Zustand, so dass es im in dem EMS-Kleidungsstück eingenähten oder angebrachten Zustand des EMS-Reizstromübertragungselements bereits als rückseitige Isolierung der EMS-Elektrode bzw. deren Stromübertragungsbereichs dienen kann. Alternativ dazu kann das EMS-Kleidungsstück entsprechende Isolationsstrukturen aufweisen, die das EMS-Reizstromübertragungselement im stromführenden Bereich nach außen hin abdecken.

Um dem EMS-Reizstromübertragungselement insgesamt, aber besonders im von den Stromübertragungsbereichen und den Zuleitungen erfassten Abschnitt die gewünschte Elastizität zu erhalten, die mit einem textilen Trägermaterial erzielt werden kann, z:B. unter Verwendung von Elastan, können die die einzelnen Stromübertragungsbereiche samt Zuleitung ausbildenden Stromleiterstränge jeweils einer Zick-Zack-Bahn folgend oder mäanderierend an dem Trägerelement befestigt, also vorteilhaft aufgenäht oder aufgestickt sein. Kommt Zug auf den entsprechenden Stromleiterstrangabschnitt, so kann er wie eine Ziehharmonika nachgeben. Dabei kann es ausreichen, wenn nur die voraussichtlich zugbelasteten Stromleiterstrangabschnitte zumindest abschnittsweise einer Zick-Zack-Bahn folgend oder mäanderierend verlegt sind. So könnten die- bzw. derjenige Stromleiterstrangabschnitt, der von dem Stromübertragungsbereich zur beabstandeten Anschlussstelle bzw. Masseanschlussstelle führt, den Zick-Zack-Verlauf haben.

Schon um den vorzugsweise einzigen Stromleiterstrang im Stromübertragungsbereich flächig zu verlegen wird der Stromleiterstrang im Stromübertragungsbereich insgesamt einer Zick-Zack-Bahn folgend mit einer Mehrzahl in elektrischer Reihenschaltung aufeinander folgender, parallel nebeneinander angeordneter Stromleiterstrangabschnitte verlegt werden. Damit ergibt sich nicht nur eine gute Abdeckung der Fläche des Stromübertragungsbereichs, sondern gleichzeitig eine Dehnbarkeit des Stromleiterstrangs und des Trägertextils im Stromübertragungsbereich quer zur Richtung, in die die Stromleiterstrangabschnitte dort verlegt sind.

Um auch noch eine entsprechende Elastizität bzw. Dehnbarkeit in der Richtung zu erhalten, in der die die Stromleiterstrangabschnitte im Stromübertragungsbereich verlaufen, können die einzelnen Stromleiterstrangabschnitte wiederum jeweils einer eigenen, gegenüber der Gesamt-Zick-Zack-Bahn im Stromübertragungsbereich entsprechend mit deutlich kleineren Amplituden verlaufenden Zick-Zack-Bahn folgend bzw. mäanderierend verlegt sein. Zum Verlegen der Stromleiterstrangabschnitte im Stromübertragungsbereich können diese auf das textile Trägerelement aufgestickt und damit lagefixiert werden.

Über die Aufstickformen (mäandrierende Bahn, Gerade, großflächige Mäanderbahn mit geraden oder mäandrierenden Bahnabschnitten) lassen sich also verschiedene Eigenschaften des EMS-Reizstromübertragungselements erzeugen, wobei hierbei auch immer entscheidend ist, dass das Trägermaterial, dass für den jeweiligen Einsatzzweck verwendet werden soll, dazu passt. So kann über eine großflächige Mäanderbahn eine Fläche eines Stromübertragungsbereichs einer EMS-Elektrode erzeugt bzw. abgedeckt werden, durch Geraden können z.B. Stromleiterbahnabschnitte umgesetzt werden, die keine Elastizität zulassen sollen (z.B. an der Zuleitung von der Anschlussstelle zum Stromübertragungsbereich), und mittels mäanderförmig aufgestickter Stromleiterbahnabschnitte kann diesen Stromleiterbahnabschnitten ein gewisses Maß an Flexibilität und Elastizität ermöglicht werden. Zusätzlich kann über die mäanderförmige bzw. Zick-Zack-Bahn eine maximale Zugbegrenzung festgelegt werden, die vor dem Erreichen der maximalen Dehngrenze des Trägermaterials eintritt. D.h., die mäanderförmigen Stromleiterbahnabschnitte erreichen ihre maximale Streckgrenze vor der maximalen Dehngrenze des Trägermaterials und ggfs. weiterer eingearbeiteter elastischer Materialien, so dass ein Reißen des Trägermaterials und ggfs. weiterer eingearbeiteter elastischer Materialien verhindert wird. Hierdurch wird die Haltbarkeit des Trägermaterials und ggfs. Der weiteren eingearbeiteten elastischer Materialien deutlich erhöht.

Der Stromleiterstrang bzw. die Stromleiterstränge können als Einzelader ausgeführt sein, z.B. aus einem Einzelmetalldraht bestehen oder aus einer metallbeschichteten Kunststofffaser. Derartige Einzeladern sind aber in puncto Biegsamkeit, sowie Flexibilität und Dehnbarkeit des mit der Einzelader verbundenen Trägertextils gegenüber Faserverbunden im Nachteil. Vorteilhaft ist daher jeder Stromleiterstrang durch einen Verbund von Einzelfasern ausgebildet.

Dabei wäre grundsätzlich die Verwendung eines Geflechts, vorzugsweise Litzengeflecht oder Schlauchgeflecht als Stromleiterstrang denkbar, wobei das Geflecht Einzeladern aus Metall oder mit Metallbeschichtung aufweisen oder aus solchen Einzeladern bestehen könnte. Grundsätzlich denkbar wäre es auch, dass der Stromleiterstrang bzw. die Stromleiterstränge oder zumindest einer der Stromleiterstränge aus einem Gewebeband oder Wirkband besteht, welches Einzeladern aus Metall oder mit Metallbeschichtung aufweist oder aus solchen Einzeladern besteht.

Als vorteilhaft hat es sich jedoch herausgestellt, wenn der Stromleiterstrang aus einem Einfachgarn, gefachtem Garn, Zwirn, Kordel oder Seil besteht, welches Einzeladern aus Metall oder mit Metallbeschichtung aufweist oder aus solchen Einzeladern besteht, oder aus einer Metalllitze, deren Einzeladern von verdrillten oder verzwirnten Metalldrähten oder -fasern gebildet wird oder solche Metalldrähte oder -fasern aufweist.

Einige oder alle Einzeladern bzw. Einzelfasern im Verbund des Stromleiterstrangs könnten beispielsweise als Folienbändchen aus metallbeschichteter Folie ausgebildet sein. Als geeigneter haben sich jedoch als Filament oder Spinnfaser ausgebildete Einzeladern herausgestellt, insbesondere Filamente, also Mono- oder Multifilamente aus Metall oder aus metallbeschichtetem Kunststoff.

In Versuchen hat sich insbesondere ein Multifilamentgarn aus Edelstahlmultifilamenten als zum einen kostengünstig, zum anderen flexibel, einfach zu verlegen und im Hinblick auf die Stromleitereigenschaften ausreichend herausgestellt. Neben Edelstahl eignen sich aber prinzipiell auch weitere Materialien wie Silber, verschiedene Cu-Legierungen und Silberlegierungen für die Einzelfasern. Auch Mischungen von aus unterschiedlichen Materialien bestehenden Einzelfasern im Faserverbund des Stromleiterstrangs wären denkbar.

Die einzelnen Monofilamente können dabei einen Durchmesser im Mikrometerbereich aufweisen, das Multifilamentgarn einen Durchmesser im Zehntelmillimeter bis einstelligen Millimeterbereich.

Denn im Hinblick auf eine einfache Verlegbarkeit, aber auch auf einen geringen Leitungswiderstand sind die als Stromleiterstrang verwendeten Faserverbunde dabei im Durchmesser nicht zu gering dimensioniert, weisen also beispielsweise gegenüber der in dem textilen Trägerelement verwendeten Fäden einen um eine oder mehrere Größenordnungen größeren Durchmesser auf. Gerade bei solchen relativ dicken Stromleitersträngen kommt die hohe Biegefähigkeit der Faserbunde zum Tragen.

Als besonders geeignet für den erfindungsgemäßen Einsatz im EMS-Bereich hat sich beispielsweise ein Garn, insbesondere Edelstahlgarn mit einem Durchmesser im Bereich von 0,3 - 2 mm, beispielsweise 0,75 - 1,05 mm erwiesen, insbesondere ein aus mehreren, vorzugsweise vier Einzelgarnen verdrilltes oder verzwirntes Edelstahl-Multifilamentgarn, bei dem die Einzelgarne aus jeweils 200 bis 300, z.B. 275 aus Edelstahl bestehenden Filamenten mit einem Durchmesser von 10 -15 µm, insbesondere 12 µm bestehen, wobei als Edelstahl eine Chrom-, Nickel-, Molybdän-Legierung wie z.B. WNr. 1.4435 (X2CrNiMo18-14-3) zum Einsatz kommen kann, die den zusätzlichen Vorteil aufweist, dass sie nicht magnetisierbar ist.

Der als Stromleiterstrang verwendete Faserverbund, insbesondere das vorstehend erläuterte Garn kann ferner zum Korrosionsschutz eine Beschichtung aufweisen. Geeignet hierfür hat sich eine PFA- bzw. Perfluoralkoxy-Beschichtung erwiesen. Die Korrosionsschutz-Beschichtung trägt dazu bei, die vorstehend beschriebene Wirkkette von durch Korrosion verursachten Widerstandsunterschieden, die wiederum zu verstärkter Korrosion führen, weiter zu unterdrücken.

Gemäß einer weiteren vorteilhaften Weiterbildung ist der bzw. sind die zwischen der Anschlussstelle bzw. Masseanschlussstelle und dem Stromübertragungsbereich verlaufenden Stromleiterstrangabschnitte zwischen dem Trägerelement und einer Deckschicht verlegt. Die Deckschicht kann wie das Trägerelement ein textiles Flächengebilde sein. Ist die Deckschicht auch im genässten Zustand nicht oder zumindest nur schlecht elektrisch leitend, so kann sie als Abdeckung der stromführenden Zuleitungen zum Körper hin dienen. Des weiteren ermöglicht die Deckschicht eine einfache Verlegung des Stromleiterstrangabschnitts, indem dieser zwischen der Deckschicht und dem Trägerelement eingenäht wird. Mit anderen Worten, die Deckschicht und das Trägerelement sind durch Nahten zu beiden Seiten des Stromleiterstrangabschnitts miteinander verbunden, so dass der Stromleiterstrangabschnitt in der so geschaffenen "Röhre" lose verlegt oder noch durch Aufsticken weiter fixiert sein kann.

Vorteilhaft kann die Deckschicht das gesamte Trägerelement abdecken, bis auf den Stromübertragungsbereich. Dazu weist die Deckschicht vorteilhaft eine den Stromübertragungsbereich freilegende Öffnung auf. Dabei können das Trägerelement und die Deckschicht entlang des Rands der Öffnung miteinander vernäht, verklebt und/oder verschweißt sein. Vorteilhaft werden die Stromleiterstrangabschnitte, also bevorzugt der einzige Stromleiterstrang im Stromübertragungsbereich am Rand des Stromübertragungsbereichs von der Naht bzw. Klebe- oder Schweißstelle erfasst, mit der das Trägerelement und die Deckschicht vernäht sind.

Im in ein EMS-Kleidungsstück eingenähten, eingeklebten und/oder eingeschweißten Zustand das EMS-Reizstromübertragungselements oder am EMS-Reizstromübertragungselement selbst kann selbstverständlich auch eine den Stromübertragungsbereich abdeckende Schicht, z.B. textile Schicht vorgesehen sein, so dass der Stromfluss nicht direkt auf den menschlichen oder tierischen Körper erfolgt, sondern durch diese Decklage hindurch. Insbesondere wäre dafür eine gut wasserspeichernde, nichtisolierende Schicht mit guter Anpassung an die Körperform denkbar (Schwamm, Mikrofaservliess, Hochflor etc.).

Wie eingangs erwähnt sind heutige EMS-Kleidungsstücke meist mit einer Vielzahl von EMS-Elektrodenpaaren versehen, um eine Vielzahl von Muskelgruppen zu trainieren, wobei jedem Muskelabschnitt ein Paar einander entsprechende EMS-Elektroden zugeordnet ist, die sich in einem von der EMS-Reizerzeugungseinheit zum Körper erstreckende Leitungsast und in einem vom Körper zurück erstreckenden Leitungsast befinden, so dass ein Stromkreis durch den jeweiligen Muskelabschnitt gebildet wird. Das EMS-Reizstromübertragungselement weist daher vorteilhaft eine geradzahlige Mehrzahl von Leistungsästen auf, von denen jeder einen Stromübertragungsbereich und dessen Anschlussstromleiterstrangabschnitt(e) umfasst, welche wie vorstehend erläutert jeweils durch die Stromleiterstrangabschnitte eines einzigen Stromleiterstrangs gebildeten Stromübertragungsbereich, durch nebeneinander verlegte Stromleiterstrangabschnitte parallel geschalteter Stromleiterstränge oder durch aufspleißen eines Stromleiterstrangs zu einzelnen Stromleiterstrangabschnitten gebildet wird. Mit anderen Worten, jeder der Leitungsäste wird von einem einzigen, im Stromübertragungsbereich flächig verlegten oder aufgespleißten Stromleiterstrang oder von einer Mehrzahl parallel geschalteter, im Stromübertragungsbereich parallel verlegter Stromleiterstränge gebildet. Dabei sind jeweils zwei der Leitungsäste einander zugeordnet und mit einander entsprechenden Stromübertragungsbereichen ausgestattet, um einen durch einen Muskelabschnitt des lebenden Körpers führenden Stromkreis zu bilden.

Um weitere, externe EMS-Elektroden an das EMS-Kleidungsstück anschließen zu können, beispielsweise auf Armbänder sitzende EMS-Elektroden an eine EMS-Weste weist das EMS-Reizstromübertragungselement ferner vorteilhaft eine weitere geradzahlige Mehrzahl von Zusatzleistungsästen auf, welche jeweils von einer Anschlussstelle zum Anschluss an die EMS-Reizerzeugungseinheit zu einer davon beabstandeten Elektrodenanschlussstelle zum Anschluss einer externen EMS-Elektrode führen, und welche selbst keine EMS-Elektrode enthalten.

Die Vorteile dieser Weiterbildung zeigen sich besonders deutlich, wenn die Leitungsäste und die Zusatzleistungsäste an einer gemeinsamen Anschlussstelle enden, vorzugsweise in einem gemeinsamen Stecker. Die Verkabelung sämtlicher EMS-Elektroden mit der EMS-Reizerzeugungseinheit kann dann mit einem gemeinsamen, von der EMS-Reizerzeugungseinheit zu der Anschlussstelle am EMS-Kleidungsstück bzw. am dortigen Stecker führenden Kabel erfolgen.

Zur Kontaktierung am Stecker können dabei die Litzen bzw. Einzelfasern am Ende der Leitungsäste aufgedröselt und in dem Stecker verlötet, steckverbunden oder vergossen werden. Ebenfalls besteht die Möglichkeit des Crimpens, Lötens oder Schraubens an ein weiteres Leitermedium. Die Ende der Litzen oder der Multifilamente könnten auch über ein Spleißverfahren in ein weiteres Leitermedium eingespleißt werden.

Wenn sich die Leitungsäste und sofern vorhanden die Zusatzleistungsäste in einem zwischen der gemeinsamen Anschlussstelle und den Stromübertragungsabschnitten befindlichen Bereich parallel und benachbart zueinander erstrecken und dann kabelbaumartig in die einzelnen Stromübertragungsabschnitte auffächern ergibt sich ein kompakter und stabiler Aufbau des EMS-Reizstromübertragungselements mit kurzen Stromleitersträngen.

In den beiliegenden Figuren werden verschiedene Ausführungsformen in der Erfindung näher erläutert. Es zeigen:
- Figur 1: eine schematische Ansicht einer EMS-Trainingsvorrichtung mit einer als EMS-Kleidungsstück gestalteten EMS-Reizstromübertragungs-element gemäß einer ersten Ausführungsform der Erfindung;
- Figur 2: eine schematische Ansicht eines als Einsatz für ein EMS-Kleidungsstück gestalteten EMS-Reizstromübertragungselements gemäß einer weiteren Ausführungsform der Erfindung;
- Figur 3: Einzelheit III in Figur 2;
- Figur 4: Einzelheit IV in Figur 2; und
- Figur 5: Einzelheit V in Figur 2;

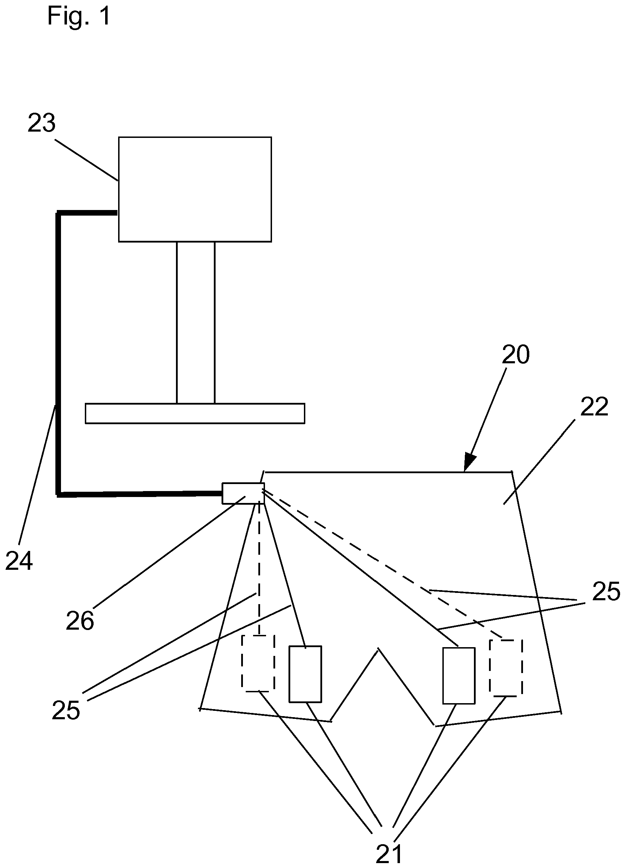

In der Figur 1 ist ein als kurze Hose gestaltetes Reizstromübertragungselement 20 gezeigt, welches mit vier jeweils zu zwei Paaren gruppierten EMS-Elektroden 21 bestückt ist, wobei jedes Elektrodenpaar einem Oberschenkel zugeordnet ist, und wobei jede EMS-Elektrode 21 über einen Stromleiterstrangabschnitt 25 mit einer beabstandeten, in einem Stecker 26 befindliche Anschlussstelle verbunden ist. Von dem Stecker 26 führt ein gemeinsames Signalkabel 24 zu einer in ein pultförmiges Steuergerät eingebauten EMS-Reizerzeugungseinheit 23. Die EMS-Reizerzeugungseinheit 23 formt aus einem Netzstrom EMS-Reize gemäß eines gewünschten Anregungsschema und beschickt damit abwechselnd die beiden EMS-Elektroden 25 jedes Elektrodenpaars.

Die EMS-Elektroden 21, die Stromleiterstrangabschnitte 25 und der Stecker 26 sind dabei auf einem textilen Trägerelement 22 angebracht. Die Stromleiterstrangabschnitte 25 von dem Stecker 26 zu den EMS-Elektroden 21 und die EMS-Elektroden 21 selbst bestehen dabei jeweils aus einem Stromleiterstrang aus Edelstahlgarn, welches wiederum aus einigen, z.B. sechs Multifilamentsträngen besteht und welches im von der zugeordneten EMS-Elektroden 21 bedeckten Bereich, dem Stromübertragungsbereich 21, in seine einzelnen Multifilamentstränge aufgetrennt und dort flächig verlegt ist. Das Edelstahlgarn ist auf das textile Trägerelement 22 aufgestickt und im Bereich der die Anschlussleitungen 25 bildenden Stromleiterstrangabschnitte 25 mit einer elektrisch isolierenden Deckschicht (nicht gezeigt) nach innen, also zum Körper hin, abgedeckt.

Das Trägerelement 22, die EMS-Elektroden 21, die Zuleitungen 25 und der Stecker 26 bilden ein EMS-Reizstromübertragungselement 20, welches bereits als EMS-Kleidungsstück 20, nämlich kurze Hose ausgeformt ist.

Das EMS-Reizstromübertragungselement könnte natürlich auch als ein anderes EMS-Kleidungsstück geformt sein, etwa als Weste oder als Ganzkörperanzug. sein. Es könnte auch ein Satz von mehreren EMS-Kleidungsstücken, z.B. Hose, Weste, Armbänder, Unterschenkelbänder vorgesehen sein.

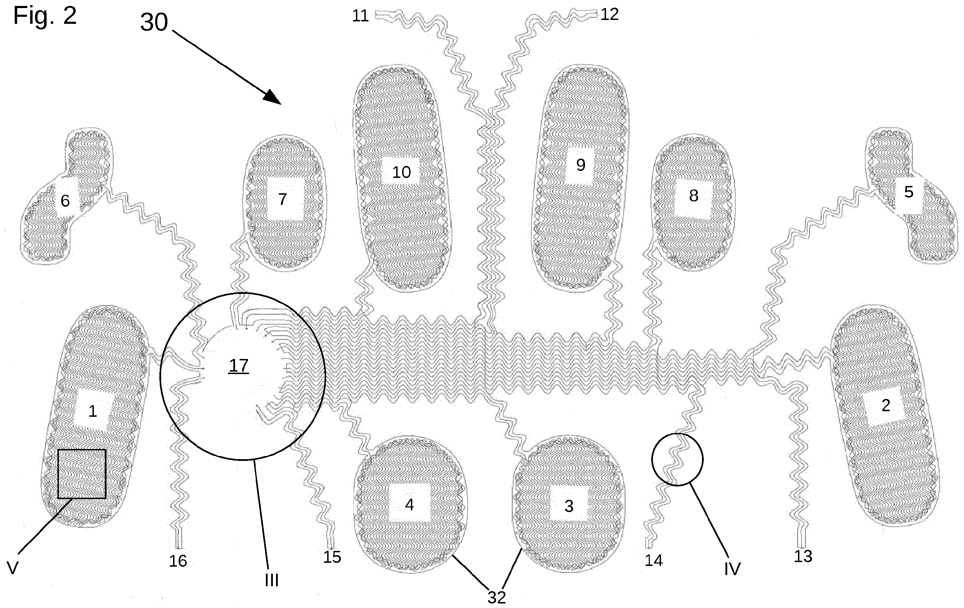

Das EMS-Reizstromübertragungselement könnte natürlich auch eine ganz andere Gestalt haben, etwa als ein in EMS-Kleidungsstück einnähbarer Einsatz, wie das bei der in Figur 2 gezeigten Ausführungsform der Erfindung der Fall ist.

Das in Figur 2 gezeigte EMS-Reizstromübertragungselement 30 ist als Einsatz zum Einnähen oder Einkleben in ein als Weste gestaltetes EMS-Kleidungsstück konzipiert und weist dazu ein aus einem aufgetrennten Rundstricktextil entlang linienförmiger Stromleiterstränge 1 - 16 ausgeschnittenes Trägerelement 32 auf. Die Stromleiterstränge 1 - 16 sind dabei vor dem Zuschnitt auf das Trägerelement 32 in der gezeigten Form aufgestickt worden.

Dabei führen die Stromleiterstränge 1 - 10 jeweils von einer gemeinsamen Anschlussstelle 17 in einen Bereich, in dem sie jeweils flächig verlegt sind. Diese Bereiche, in denen jeweils einer der Stromleiterstränge 1 - 10 flächig verlegt ist, dienen im in das EMS-Kleidungsstück eingenähten Zustand des EMS-Reizstromübertragungselements 30 jeweils als Stromübertragungsbereich einer EMS-Elektrode des EMS-Kleidungsstücks. Leitungsäste 1 - 10, welche jeweilis eine EMS-Elektrode und die Zuleitung von der Anschlussstelle bis zur EMS-Elektrode enthalten werden also jeweils von einem einzigen, vorzugsweise aus Eldelstahlmultifilamentgarn ausgebildeten, linienförmigen Stromleiterstrang 1 - 10 gebildet.

Jeweils zwei EMS-Elektroden sind dabei einander zugeordnet, wobei deren Stromübertragungsbereiche einander größenmäßig entsprechen, etwa die Stromübertragungsbereiche in den Leitungsästen 9 und 10. Die beiden einander zugeordneten Stromübertragungsbereiche befinden sich entsprechend in zwei der Leitungsäste 1 - 10, die einander zugeordnet sind, etwa in den Leitungsästen 9 und 10 und bilden während des EMS-Trainings einen Stromkreis durch den zu trainierenden Muskelabschnitt des Körpers, im Falle der Leitungsäste 9 und 10 etwa die rückseitigen Rumpfmuskelpartien.

Weitere Zusatzleitungsäste 11 - 16, welche ebenfalls von einem einzigen, linienförmigen Stromleiterstrang, nämlich aus Garn gebildet werden, beginnen ebenfalls an der gemeinsamen Anschlussstelle 17 und führen zu davon entfernten, freien Enden. Dort können zusätzliche EMS-Elektroden für die Extremitäten angeschlossen werden können, etwa bei 15 und 16 ein EMS-Elektroden-Paar für den einen Bizeps, bei 13 und 14 ein EMS-Elektroden-Paar für den anderen Bizeps. Die Verkabelung der zusätzlichen, externen EMS-Elektroden mit der EMS-Reizerzeugungseinheit kann dann ebenfalls über einen an der gemeinsamen Anschlussstelle 17 anzubringenden Stecker erfolgen.

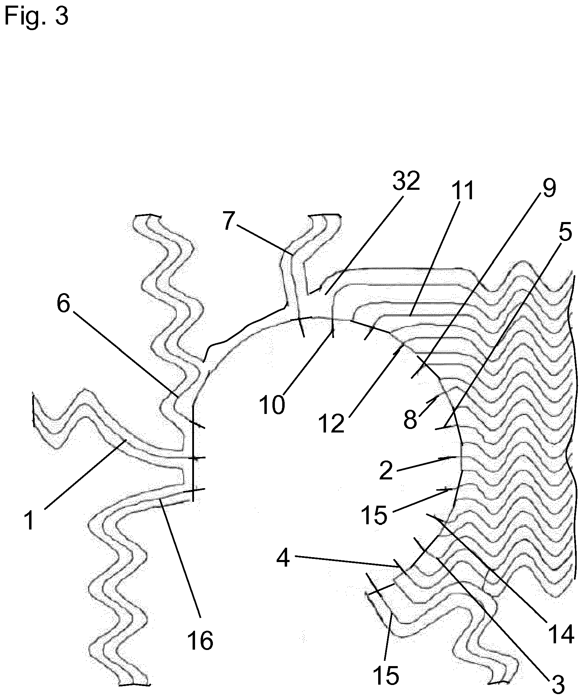

Die gemeinsame Anschlussstelle 17 ist in Figur 3 im Einzelnen gezeigt. Man erkennt die Enden der Stromleiterstränge 1 - 16, die beispielsweise über ein Spleißverfahren mit einem Stecker verbunden werden können. Während die Stromleiterstränge 1, 6, 7, 15 und 16 sich dort sofort auf separaten Abschnitten des Trägerelements 32 erstrecken, die nur im Bereich der gemeinsamen Anschlussstelle 17 mit dem Rest des Trägerelements 32 verbunden sind, erstrecken sich die anderen Stromleiterstränge 2 - 5, 8 - bis 14 zunächst in einem gemeinsamen Bereich und fächern dann kabelbaumartig in einzelne Äste auf.

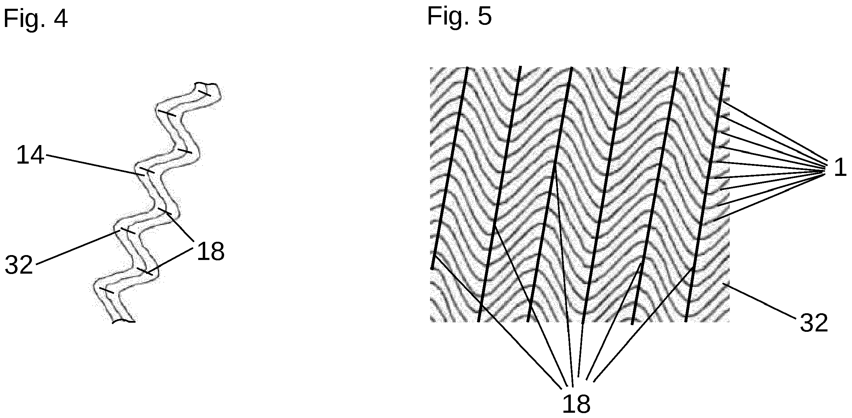

Die Stromleiterstränge 1 - 16 sind dabei in ihrem gesamten Verlauf (bis auf kurze Zuleitungen an der gemeinsamen Anschlussstelle 17) mäandrierend bzw. zick-zack-bahnförmig auf das Trägerelement 32 aufgestickt, wie insbesondere aus den Figuren 4 und 5 hervorgeht, in denen die Sticknähte jeweils mit Bezugsziffer 18 bezeichnet sind.

Abwandlungen und Modifikationen der gezeigten Ausführungsformen sind möglich, ohne den Rahmen der Erfindung zu verlassen.

## Patentansprüche

1. EMS-Reizstromübertragungselement (20; 30) für ein EMS-Kleidungsstück (20), mit zumindest einem flächenförmigen Stromübertragungsbereich (21) einer EMS-Elektrode zur Übertragung von EMS-Reizen auf den lebenden Körper, welcher eine Anzahl flächig angeordneter, linienförmiger Stromleiterstrangabschnitte enthält und über eine weitere Anzahl linienförmiger Stromleiterstrangabschnitte (25) an eine vom Stromübertragungsbereich (21) beabstandete Anschlussstelle (17; 26) angeschlossen ist, an der das EMS-Reizstromübertragungselement (20; 30) an eine EMS-Reizerzeugungseinheit (23) anschließbar ist, um den Stromübertragungsbereich (21) mit dem von der EMS-Reizstromerzeugungseinheit (23) aus einem von einer Stromquelle bezogenen Strom zu einer Impulsfolge und/oder zu einem Wechselstrom geformten EMS-Reizstrom zu beschicken,
**dadurch gekennzeichnet, dass**
die EMS-Elektrode durch einen einzigen, linienförmigen Stromleiterstrangabschnitt ausgebildet wird, der im Stromübertragungsbereich ohne Maschen flächig verlegt ist und mitsamt dem zur Anschlussstelle (17) führenden Stromleiterstrangabschnitt einen einzigen, linienförmigen Stromleiterstrang (1 - 10) bildet, so dass der Stromfluss dem linienförmigen Stromleiterstrang (1 - 10) folgt und nicht von Masche zu Masche bzw. in einem Gewebe springt,
oder dass
die EMS-Elektrode anstatt durch den einzigen, linienförmigen Stromleiterstrangabschnitt im Stromübertragungsbereich durch eine Mehrzahl linienförmiger, im Stromübertragungsbereich parallel und jeweils ohne Maschen flächig verlegter Stromleiterstrangabschnitte ausgebildet wird, die jeweils mit einem der zur Anschlussstelle führenden Stromleiterstrangabschnitte einen einzigen, linienförmigen Stromleiterstrang bilden,
oder dass
die EMS-Elektrode anstatt durch den einzigen, linienförmigen Stromleiterstrangabschnitt im Stromübertragungsbereich durch eine Mehrzahl linienförmiger, im Stromübertragungsbereich (21) parallel und ohne Maschen flächig verlegter Stromleiterstrangabschnitte ausgebildet wird, die alle mit dem einzigen, linienförmigen Stromleiterstrangabschnitt (25) verspleißt sind, der zur Anschlussstelle (26) führt.

2. EMS-Reizstromübertragungselement nach Anspruch 1, **dadurch gekennzeichnet, dass** der Stromübertragungsbereich an eine vom Stromübertragungsbereich insbesondere beabstandete, weitere Anschlussstelle angeschlossen ist, an der das EMS-Reizstromübertragungselement an die EMS-Reizerzeugungseinheit anschließbar ist, wobei
der Stromübertragungsbereich über einen einzigen Stromleiterstrangabschnitt an die weitere Anschlussstelle angeschlossen ist, der an dem selben einzigen, linienförmigen Stromleiterstrang ausgebildet ist wie der einzige, flächig verlegte, Stromleiterstrangabschnitt im Stromübertragungsbereich,
oder wobei
der Stromübertragungsbereich über eine Mehrzahl von Stromleiterstrangabschnitten an die weitere Anschlussstelle angeschlossen ist, von denen jeder mit einem der im Stromübertragungsbereich parallel verlegten Mehrzahl linienförmiger Stromleiterstrangabschnitte an einem einzigen, linienförmigen Stromleiterstrang ausgebildet ist,
oder wobei
der Stromübertragungsbereich über einen einzigen Stromleiterstrangabschnitt an die weitere Anschlussstelle angeschlossen ist, in welchen alle der im Stromübertragungsbereich parallel verlegten Mehrzahl linienförmiger Stromleiterstrangabschnitte eingespleißt sind.

3. EMS-Reizstromübertragungselement (20; 30) nach Anspruch 1 oder 2, **dadurch gekennzeichnet, dass** die Stromleiterstrangabschnitte, bevorzugt der einzige Stromleiterstrang auf einem vorteilhaft als textiles Flächengebilde (20; 32) ausgebildeten Trägerelement (20; 32) angeordnet sind bzw. ist.

4. EMS-Reizstromübertragungselement (20; 30) nach Anspruch 3, **dadurch gekennzeichnet, dass** die Stromleiterstrangabschnitte, bevorzugt der einzige Stromleiterstrang zumindest im Bereich des Stromübertragungsabschnitts auf das Trägerelement (20; 32) aufgenäht oder aufgestickt sind bzw. ist.

5. EMS-Reizstromübertragungselement (20; 30) nach Anspruch 3 oder 4, **dadurch gekennzeichnet, dass** das Trägerelement (20; 32) nicht elektrisch leitend ist,

6. EMS-Reizstromübertragungselement (30) nach einem der vorhergehenden Ansprüche, **dadurch gekennzeichnet, dass** zumindest einer der Stromleiterstrangabschnitte zumindest abschnittsweise einer Zick-Zack-Bahn folgend oder mäandrierend verlegt, vorzugsweise an dem Trägerelement (32) befestigt ist.

7. EMS-Reizstromübertragungselement (20; 30) nach einem der vorhergehenden Ansprüche, **dadurch gekennzeichnet, dass** jeder Stromleiterstrang (1 - 10) durch einen Verbund von Einzelfasern ausgebildet ist, wobei jeder Stromleiterstrang (1 - 10) aus einem Einfachgarn, gefachtem Garn, Zwirn, Kordel oder Seil besteht, welches Einzeladern aus Metall oder mit Metallbeschichtung aufweist oder aus solchen Einzeladern besteht, oder aus einer Metalllitze, deren Einzeladern von verdrillten oder verzwirnten Metalldrähten oder -fasern gebildet wird oder solche Metalldrähte oder -fasern aufweist.

8. EMS-Reizstromübertragungselement (20; 30) nach Anspruch 7, **dadurch gekennzeichnet, dass** jeder Stromleiterstrang (1 - 10) als Filament oder Spinnfaser ausgebildete Einzeladern aufweist oder aus solchen Einzeladern besteht, insbesondere als Mono- oder bevorzugt Multifilamente aus Metall oder aus metallbeschichtetem Kunststoff ausgebildete Einzelfasern aufweist oder aus solchen Einzelfasern besteht.

9. EMS-Reizstromübertragungselement (30) nach Anspruch 6, 7 oder 8, **dadurch gekennzeichnet, dass** der Stromübertragungsbereich eine Mehrzahl parallel nebeneinander verlegter, an dem Trägerelement (32) befestigter und jeweils einer Zick-Zack-Bahn folgender oder mäanderierender Stromleiterstrangabschnitte aufweist.

10. EMS-Reizstromübertragungselement (30) nach einem der Ansprüche 6 bis 9, **dadurch gekennzeichnet, dass** die im Stromübertragungsbereich parallel nebeneinander verlegten Stromleiterstrangabschnitte in elektrischer Reihenschaltung aufeinander folgend insgesamt einen zick-zack-bahnförmigen Abschnitt des einzigen Stromteiterstrangs bilden.

11. EMS-Reizstromübertragungselement (20; 30) nach einem der Ansprüche 3 bis 10, **dadurch gekennzeichnet, dass** der bzw. die zwischen der Anschlussstelle und dem Stromübertragungsbereich verlaufenden Stromleiterstrangabschnitte zwischen dem Trägerelement (20; 32) und einer vorzugsweise textilen Deckschicht verlegt sind, wobei die Deckschicht bevorzugt nicht elektrisch leitend ist.

12. EMS-Reizstromübertragungselement (20; 30) nach Anspruch 11, **dadurch gekennzeichnet, dass** die Deckschicht eine den Stromübertragungsbereich freilegende Öffnung aufweist, wobei das Trägerelement (20; 32) und die Deckschicht entlang des Rands der Öffnung miteinander vernäht sind, und wobei sich die Stromleiterstrangabschnitte, bevorzugt der einzige Stromleiterstrang am Rand des Stromübertragungsbereichs zwischen dem Trägerelement (20; 32) und der Deckschicht erstrecken,

13. EMS-Reizstromübertragungselement (20; 30) nach einem der vorhergehenden Ansprüche, **dadurch gekennzeichnet, dass** das EMS-Reizstromübertragungselement (20; 30) eine geradzahlige Mehrzahl von Leistungsästen (21, 25; 1 - 10) aufweist, von denen jeder einen Stromübertragungsbereich (21) und dessen Anschlussstromleiterstrangabschnitt(e) (25) umfasst, wobei jeder der Leitungsäste (21, 25; 1 - 10) von einem einzigen, im Stromübertragungsbereich (21) flächig verlegten (1 - 10) oder aufgespleißten (25) Stromleiterstrang (21, 25; 1 - 10) oder einer Mehrzahl, im Stromübertragungsbereich parallel verlegter Stromleiterstränge gebildet wird, wobei jeweils zwei der Leitungsäste (21, 25; 1 - 10) einander zugeordnet und mit einander entsprechenden Stromübertragungsbereichen (21) ausgestattet sind, um einen durch einen Muskelabschnitt des lebenden Körpers führenden Stromkreis zu bilden.

14. EMS-Reizstromübertragungselement (20; 30) nach Anspruch 13, **dadurch gekennzeichnet, dass** das EMS-Reizstromübertragungselement (20; 30) eine weitere geradzahlige Mehrzahl von Zusatzleistungsästen (11 - 16) aufweist, welche jeweils von einer Anschlussstelle zum Anschluss an die EMS-Reizerzeugungseinheit zu einer davon beabstandeten Elektrodenanschlussstelle zum Anschluss einer externen EMS-Elektrode führen, und welche selbst keine EMS-Elektrode enthalten.

15. EMS-Reizstromübertragungselement (20; 30) nach Anspruch 13 oder 14, **dadurch gekennzeichnet, dass** die Leitungsäste (21, 25; 1 - 10) und sofern vorhanden die Zusatzleistungsäste (11 - 16) an einer gemeinsamen Anschlussstelle (26; 17) enden, vorzugsweise in einem gemeinsamen Stecker (26),

16. EMS-Reizstromübertragungselement (30) nach Anspruch 15, **dadurch gekennzeichnet, dass** sich die Leitungsäste (1 - 10) und sofern vorhanden die Zusatzleistungsäste (11 - 16) in einem zwischen der gemeinsamen Anschlussstelle (17) und den Stromübertragungsbereichen befindlichen Bereich parallel und benachbart zueinander erstrecken und dann kabelbaumartig in die einzelnen Stromübertragungsabschnitte auffächern.

17. EMS-Kleidungsstück (20), **dadurch gekennzeichnet, dass** es zumindest ein EMS-Reizstromübertragungselement (20; 30) nach einem der vorhergehenden Ansprüche aufweist oder von einem solchen EMS-Reizstromübertragungselement (20) gebildet wird.

## Claims

1. An EMS stimulation current transmission element (20; 30) for an EMS garment (20) with at least one planar current transmission region (21) of an EMS electrode for transmitting EMS stimuli to the living body, which current transmission region (21) contains a number of two-dimensionally arranged linear current conductor strand sections and is connected, via a further number of linear current conductor strand sections (25), to a connection point (17; 26) that is spaced apart from the current transmission region (21), at which connection point (17; 26) the EMS stimulation current transmission element (20; 30) can be connected to an EMS stimulation production unit (23) in order to load the current transmission region (21) with an EMS stimulation current shaped by the EMS stimulation current production unit (23) from a current drawn from a current source to form a pulse sequence and/or to form an alternating current,
**characterized in that**
the EMS electrode is formed by a single linear current conductor strand section, which is laid within the current transmission region in a two-dimensional manner without meshes, and together with the current conductor strand section leading to the connection point (17) is formed by a single linear current conductor strand (1 - 10), such that the current flow follows the linear current conductor strand (1 - 10) and does not jump from mesh to mesh and/or in the fabric,
or that
the EMS electrode is formed, instead of being formed by the single linear current conductor strand section in the current transmission region, by a plurality of linear current conductor strand sections laid within the current transmission region in a two-dimensional manner and in parallel and without meshes and which each form, together with one of the current conductor strand sections leading to the connection point, a single linear current conductor strand,
or that
EMS electrode is formed, instead of being formed by the single linear current conductor strand section in the current transmission region, by a a plurality of linear current conductor strand sections laid within the current transmission region (21) in a two-dimensional manner and in parallel and without meshes, which current conductor strand sections are all spliced with the single linear current conductor strand section (25) which leads to the connection point (26).

2. The EMS stimulation current transmission element according to claim 1, **characterized in that** the current transmission region is connected to a further connection point in particular spaced apart from the current transmission region, on which connection point the EMS stimulation current transmission element can be connected to the EMS stimulation production unit, wherein
the current transmission region via a single current conductor strand section is connected to the further connection point, which is formed on the same single linear current conductor strand as the single current conductor strand section laid in a two-dimensional manner in the current transmission region,
or wherein
the current transmission region via a plurality of current conductor strand sections is connected to the further connection point, of which each one is formed on a single linear current conductor strand with one of the plurality of linear current conductor strands laid in parallel in the current transmission region,
or wherein
the current transmission region via a single current conductor strand section is connected to the further connection point, into which all of the plurality of linear current conductor strand sections laid in parallel in the current transmission region are spliced.

3. The EMS stimulation current transmission element (20; 30) according to claim 1 or 2, **characterized in that** the current conductor strand sections, preferably the single current conductor strand, is and/or are arranged on a carrier element (20; 32) formed advantageously as a textile flat fabric (20; 32).

4. The EMS stimulation current transmission element (20; 30) according to claim 3, **characterized in that** the current conductor strand sections, preferably the single current conductor strand, at least in the region of the current transmission section, is sewn or embroidered onto the carrier element (20; 32).

5. The EMS stimulation current transmission element (20; 30) according to claim 3 or 4, **characterized in that** the carrier element (20; 32) is not electrically conductive.

6. The EMS stimulation current transmission element (30) according to any one of the preceding claims, **characterized in that** at least one of the current conductor strand sections at least in sections is laid following a zigzag track or in a meandering manner, preferably is attached to the carrier element (32).

7. The EMS stimulation current transmission element (20; 30) according to any one of the preceding claims, **characterized in that** each current conductor strand (1 - 10) is formed by a composite of single fibers, wherein each current conductor strand (1 - 10) consists of a single yarn, doubled yarn, twist, cord or rope which comprises single conductors made out of metal or with metal coating or consists of such single conductors or of a metal strand the single conductors of which are formed by twisted or plied metal wires or metal fibers or comprises such metal wires or metal fibers.

8. The EMS stimulation current transmission element (20; 30) according to claim 7, **characterized in that** each current conductor strand (1 - 10) comprises single conductors formed as a filament or as a staple fiber, or consists of such single conductors, in particular comprises single fibers formed as monofilaments or preferably multifilaments made out of metal or out of metal-coated plastics, or consists of such single fibers.

9. The EMS stimulation current transmission element (30) according to claim 6, 7 or 8, **characterized in that** the current transmission region comprises a plurality of current conductor strand sections laid in parallel next to each other, attached to the carrier element (32) and each following a zigzag track or in a meandering manner.

10. The EMS stimulation current transmission element (30) according to any one claims 6 to 9, **characterized in that** the current conductor strand sections laid in parallel next to each in the current transmission region in an electrical serial connection successively form in total a zigzag track section of the single current conductor strand.

11. The EMS stimulation current transmission element (20; 30) according to any one claims 3 to 10, **characterized in that** the current conductor strand section(s) extending between the connection point and the current transmission region are laid between the carrier element (20; 32) and a preferably textile top layer, wherein the top layer preferably is not electrically conductive.

12. The EMS stimulation current transmission element (20; 30) according to claim 11, **characterized in that** the top layer comprises an opening exposing the current transmission region, wherein the carrier element (20; 32) and the top layer are sewn with each other along the edge of the opening, and wherein the current conductor strand sections, preferably the single current conductor strand, extend(s) on the edge of the current transmission region between the carrier element (20; 32) and the top layer.

13. The EMS stimulation current transmission element (20; 30) according to any one of the preceding claims, **characterized in that** the EMS stimulation current transmission element (20; 30) comprises an even plurality of line branches (21, 25; 1 - 10) of which each one comprises a current transmission region (21) and its connecting current conductor strand section(s) (25) wherein each of the line branches (21, 25; 1 - 10) is formed by a single current conductor strand (21, 25; 1 - 10) laid in a two-dimensional manner (1 - 10) in the current transmission region (21) or by a current conductor strand (21, 25; 1 - 10) spliced open, or by a plurality of current conductor strands laid in parallel in the current transmission region, wherein two of the line branches (21, 25; 1 - 10) each are allocated to each other and equipped with current transmission regions (21) corresponding to each other in order to form an electric circuit leading through a muscle section of the living body.

14. The EMS stimulation current transmission element (20; 30) according to claim 13, **characterized in that** the EMS stimulation current transmission element (20; 30) comprises another even plurality of additional line branches (11 - 16) which lead in each case from a connection point for connection to the EMS stimulation production unit to an electrode connection point spaced apart for connection to an external EMS electrode, and which do not contain an EMS electrode themselves.

15. The EMS stimulation current transmission element (20; 30) according to claim 13 or 14, **characterized in that** the line branches (21, 25; 1 - 10), and if applicable, the additional line branches (11 - 16) end on a common connection point (26; 17), preferably in a common connector (26).

16. The EMS stimulation current transmission element (30) according to claim 15, **characterized in that** the line branches (1 - 10) and, if applicable, the additional line branches (1 - 16) extend in a region located between the common connection point (17) and the current transmission regions in parallel and adjacent to each other, and then fan out like a cable harness into the single current transmission sections.

17. An EMS garment (20), **characterized in that** it comprises at least an EMS stimulation current transmission element (20; 30) according to any one of the preceding claims or is formed by such an EMS stimulation current transmission element (20).

## Revendications

1. Elément de transmission de courant de stimulation EMS (20; 30) pour un vêtement EMS (20), comprenant au moins une zone de transmission de courant (21) en nappe d'une électrode EMS pour la transmission de stimuli EMS au corps vivant, qui comprend une pluralité de sections de brin de conducteur en forme de ligne agencées en nappe et qui est raccordée au moyen d'une autre pluralité de sections de brin de conducteur (25) en forme de ligne à un point de raccordement (17; 26) qui est espacé de la zone de transmission de courant (21) et sur lequel ledit élément de transmission de courant de stimulation EMS (20; 30) peut être raccordé à une unité de génération de stimuli EMS (23) afin d'alimenter la zone de transmission de courant (21) en courant de stimultion EMS formé par l'unité de génération de stimuli EMS (23), à partir d'un courant provenant d'une source de courant, en un train d'impulsions et/ou en un courant alternatif,
**caractérisé par le fait que**
ladite électrode EMS est réalisée par une seule section de brin de conducteur en forme de ligne qui est posée en nappe et sans mailles dans la zone de transmission de courant et qui forme un seul brin de conducteur (1 - 10) en forme de ligne conjointement avec la section de brin de conducteur menant vers ledit point de raccordement (17) de sorte que le flux de courant suit le brin de conducteur (1 - 10) en forme de ligne et ne saute pas d'une maille à l'autre ou bien dans un tissu, ou que
ladite électrode EMS, au lieu d'être réalisée par la seule section de brin de conducteur en forme de ligne dans la zone de transmission de courant, est réalisée par une pluralité de sections de brin de conducteur en forme de ligne qui sont posées en nappe, dans la zone de transmission de courant, parallèlement et respectivement sans mailles et qui forment chacune un seul brin de conducteur en forme de ligne conjointement avec l'une des sections de brin de conducteur menant vers le point de raccordement, ou que
ladite électrode EMS, au lieu d'être réalisée par la seule section de brin de conducteur en forme de ligne dans la zone de transmission de courant, est réalisée par une pluralité de sections de brin de conducteur en forme de ligne qui sont posées en nappe, dans la zone de transmission de courant (21), parallèlement et sans mailles et qui sont toutes épissées avec ladite seule section de brin de conducteur (25) en forme de ligne qui mène vers le point de raccordement (26).

2. Elément de transmission de courant de stimulation EMS selon la revendication 1, **caractérisé par le fait que** la zone de transmission de courant est raccordée à un autre point de raccordement, en particulier espacé de la zone de transmission de courant, sur lequel ledit élément de transmission de courant de stimulation EMS peut être raccordé à l'unité de génération de stimuli EMS, dans lequel
ladite zone de transmission de courant est raccordée via une seule section de brin de conducteur à l'autre point de raccordement, qui est réalisée sur le même seul brin de conducteur en forme de ligne que la seule section de brin de conducteur posée en nappe dans la zone de transmission de courant, ou dans lequel
ladite zone de transmission de courant est raccordée via une pluralité de sections de brin de conducteur à l'autre point de raccordement, dont chacune est réalisée sur un seul brin de conducteur en forme de ligne avec une de la pluralité de sections de brin de conducteur en forme de ligne posées parallèlement dans la zone de transmission de courant, ou dans lequel
ladite zone de transmission de courant est raccordée via une seule section de brin de conducteur à l'autre point de raccordement, dans laquelle toutes de ladite pluralité de sections de brin de conducteur en forme de ligne posées parallèlement dans la zone de transmission de courant sont épissées.

3. Elément de transmission de courant de stimulation EMS (20; 30) selon la revendication 1 ou 2, **caractérisé par le fait que** les sections de brin de conducteur, de préférence le seul brin de conducteur sont ou bien est disposé(es) sur un élément de support (20; 32) réalisé de préférence en tant que structure textile en nappe (20; 32).

4. Elément de transmission de courant de stimulation EMS (20; 30) selon la revendication 3, **caractérisé par le fait que** les sections de brin de conducteur, de préférence le seul brin de conducteur, sont ou bien est cousu(es) ou bien brodé(es) sur ledit élément de support (20; 32), au moins au niveau de la section de transmission de courant.

5. Elément de transmission de courant de stimulation EMS (20; 30) selon la revendication 3 ou 4, **caractérisé par le fait que** ledit élément de support (20; 32) n'est pas électriquement conducteur.

6. Elément de transmission de courant de stimulation EMS (30) selon l'une quelconque des revendications précédentes, **caractérisé par le fait que** l'une au moins des sections de brin de conducteur est posée, au moins par sections, de manière à suivre une voie en zigzag ou en méandre, de préférence est fixée sur l'élément de support (32).

7. Elément de transmission de courant de stimulation EMS (20; 30) selon l'une quelconque des revendications précédentes, **caractérisé par le fait que** chaque brin de conducteur (1 - 10) est réalisé par un assemblage de fibres individuelles, chaque brin de conducteur (1 - 10) se composant d'un fil simple, d'un fil assemblé, de fil retors, de ficelle ou de câble qui présente des fils individuels en métal ou ayant un revêtement métallique ou se compose de tels fils individuels, ou d'un toron de métal dont les fils individuels sont constitués par des fils ou fibres métalliques torsadé(e)s ou retordu(e)s ou présentent de tel(le)s fils ou fibres métalliques.

8. Elément de transmission de courant de stimulation EMS (20; 30) selon la revendication 7, **caractérisé par le fait que** chaque brin de conducteur (1 - 10) présente des fils individuels réalisés en tant que filament ou fibre discontinue ou se compose de tels fils individuels, en particulier présente des fibres individuelles réalisées en tant que mono- ou, de préférence, multi-filaments en métal ou en matière plastique revêtue de métal ou se compose de telles fibres individuelles.

9. Elément de transmission de courant de stimulation EMS (30) selon la revendication 6, 7 ou 8, **caractérisé par le fait que** la zone de transmission de courant comprend une pluralité de sections de brin de conducteur qui sont posées parallèlement les unes à côté des autres, sont fixées sur ledit élément de support (32) et suivent chacune une voie en zigzag ou sont agencées en méandre.

10. Elément de transmission de courant de stimulation EMS (30) selon l'une quelconque des revendications 6 à 9, **caractérisé par le fait que** les sections de brin de conducteur posées parallèlement les unes à côté des autres dans la zone de transmission de courant forment dans l'ensemble une section en forme de voie en zigzag dudit seul brin de conducteur en étant montées électriquement en série les unes à la suite des autres.

11. Elément de transmission de courant de stimulation EMS (20; 30) selon l'une quelconque des revendications 3 à 10, **caractérisé par le fait que** la ou bien les sections de brin de conducteur s'étendant entre le point de raccordement et la zone de transmission de courant sont posées entre ledit élément de support (20; 32) et une couche de couverture de préférence textile, de préférence ladite couche de couverture n'étant pas électriquement conductrice.

12. Elément de transmission de courant de stimulation EMS (20; 30) selon la revendication 11, **caractérisé par le fait que** ladite couche de couverture présente une ouverture exposant la zone de transmission de courant, ledit élément de support (20; 32) et ladite couche de couverture étant cousus entre eux suivant le bord de l'ouverture, et dans lequel les sections de brin de conducteur, de préférence le seul brin de conducteur, s'étend(ent) sur le bord de la zone de transmission de courant entre l'élément de support (20; 32) et la couche de couverture.

13. Elément de transmission de courant de stimulation EMS (20; 30) selon l'une quelconque des revendications précédentes, **caractérisé par le fait que** l'élément de transmission de courant de stimulation EMS (20; 30) présente une pluralité paire de branches de ligne (21, 25; 1 - 10) dont chacune comprend une zone de transmission de courant (21) et sa ou bien ses section(s) de brin de conducteur de raccordement (25), dans lequel chacune des branches de ligne (21, 25; 1 - 10) est constituée par un seul brin de conducteur (21, 25; 1 - 10) posé en nappe (1 - 10) dans la zone de transmission de courant (21) ou par une pluralité de brins de conducteur posés parallèlement dans la zone de transmission de courant, respectivement deux des branches de ligne (21, 25; 1 - 10) étant associées l'une à l'autre et étant équipées de zones de transmission de courant (21) correspondant l'une à l'autre, afin de former un circuit menant à travers une portion musculaire du corps vivant.

14. Elément de transmission de courant de stimulation EMS (20; 30) selon la revendication 13, **caractérisé par le fait que** l'élément de transmission de courant de stimulation EMS (20; 30) présente une autre pluralité paire de branches de ligne supplémentaires (11 - 16) qui mènent chacune depuis un point de raccordement pour le raccordement à une unité de génération de stimuli EMS vers un point de raccordement d'électrode en espacé et destiné à raccorder une électrode EMS externe, et qui, eux mêmes, ne comprennent pas d'électrode EMS.

15. Elément de transmission de courant de stimulation EMS (20; 30) selon la revendication 13 ou 14, **caractérisé par le fait que** les branches de ligne (21, 25; 1 - 10) et, si elles existent, les branches de ligne supplémentaires (11 - 16) se terminent sur un point de raccordement (26; 17) commun, de préférence dans une fiche commune (26).

16. Elément de transmission de courant de stimulation EMS (30) selon la revendication 15, **caractérisé par le fait que** les branches de ligne (1 - 10) et, si elles existent, les branches de ligne supplémentaires (11 - 16) s'étendent parallèlement et de manière à être voisines les unes des autres dans une zone se trouvant entre ledit point de raccordement (17) commun et les zones de transmission de courant, et, puis, se répartissent de type harnais de câbles en lesdites sections de transmission de courant individuelles.

17. Vêtement EMS (20), **caractérisé par le fait qu'**il comprend au moins un élément de transmission de courant de stimulation EMS (20; 30) selon l'une quelconque des revendication précédentes ou est constitué par un tel élément de transmission de courant de stimulation EMS (20).
